(19) Europäisches Patentamt
European Patent Office
Office européen des brevets

(11) Publication number: 0 089 768 B1

(12) EUROPEAN PATENT SPECIFICATION

(45) Date of publication of patent specification: 26.09.90

(51) Int. Cl.$^5$: A 61 K 31/557

(21) Application number: 83301200.8

(22) Date of filing: 07.03.83

# (54) Compositions containing prostaglandins.

(30) Priority: 22.03.82 GB 8208322

(43) Date of publication of application:
28.09.83 Bulletin 83/39

(45) Publication of the grant of the patent:
26.09.90 Bulletin 90/39

(84) Designated Contracting States:
BE CH DE FR GB IT LI NL SE

(56) References cited:
US-A-3 966 962

CHEMICAL ABSTRACTS, vol. 94, no. 12, 23rd March 1981, no. 90330a, Columbus, Ohio, USA, & JP-A-80149208

CHEMICAL ABSTRACTS, vol. 83, no. 22, 1st December 1975, no. 183331q, Columbus, Ohio, USA

(73) Proprietor: UPJOHN LIMITED
Fleming Way
Crawley Sussex (GB)

(72) Inventor: Johnson, Brian Samuel
c/o Upjohn Limited Fleming Way
Crawley Sussex (GB)

(74) Representative: Perry, Robert Edward et al
GILL JENNINGS & EVERY 53-64 Chancery Lane
London WC2A 1HN (GB)

EP 0 089 768 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention relates to compositions containing prostaglandins and, in particular, the relatively unstable prostaglandin E (PGE) compounds known to be useful for a wide variety of pharmacological purposes.

The prostaglandins are a family of cyclopentane-containing fatty acids. Typically, these cyclopentane derivatives contain two side-chains attached to adjacent positions of the cyclopentane ring in the relative *trans* configuration. In the natural prostaglandins, one side-chain is carboxyl-terminated, contains 7 carbon atoms and is attached in the α-configuration when this side-chain is drawn in the conventional manner. The other side-chain is alkyl-terminated, substituted by an α-hydroxyl group, and attached in the β configuration. The carbon atoms of the cyclopentane ring adjacent to the attachment of these carboxyl-terminated and hydroxyalkyl side-chains are substituted with oxygenated functional groups in the PGE compounds; these are oxo and hydroxyl groups, respectively, in the naturally-occurring PGE compounds. The compound prostaglandin $E_2$ ($PGE_2$) can be represented by the following formula, in which carbon atom numbering is given:

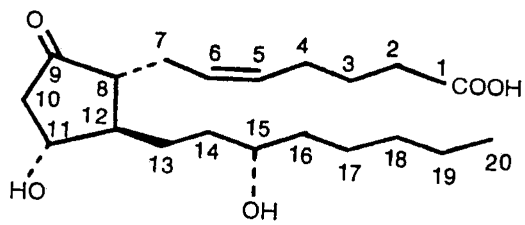

For a more complete description of PGE compounds, a relevant difinition of "prostaglandin-like compound of the PGE-type" is given in US—A—3,966,962. PGE compounds, i.e. the β-hydroxy ketones, include, for example, $PGE_2$, $PGE_1$, 15-methyl-$PGE_2$, (15R)-15-methyl-$PGE_2$, 16,16-dimethyl-$PGE_2$, 17-phenyl-18,19,20-trinor-$PGE_2$, 16-phenoxy-17,18,19,20-tetranor-$PGE_2$, 15-cyclohexyl-16,17,18,19,20-pentanor-$PGE_2$ and (11S),20-dimethyl-$PGE_2$.

The use of organic solvents, especially dry organic solvents, as in stabilising PGE compounds is known. For example, US—A—3966962 discloses the use of glyceryl triacetate or triacetin, and US—A—3829579 if dipolar aprotic solvents such as N,N-dimethylacetamide.

Protic organic solvents are also known stabilisers of PGE compounds; US—A—3749800 discloses the use of alcohols and glycols, and US—A—3927197 of tertiary alcohols.

US—A—4211793 discloses that triethyl citrate can stabilise PGE compounds. Hydrophilic gels, especially starchy gels which stabilise PGE compounds embedded therewithin, are known prostaglandin stabilisers; US—A—3816393 discloses cyclodextrin clathrates, especially β-cyclodextrin clathrates, for this purpose.

Calder *et al*, Brit. J. Obstet. Gyn. *84* (1977) 264—268, describe the use of hydroxyethyl methylcellulose as a gelling agent for $PGE_2$. The use of gelled cellulose as a stabilising agent for $PGE_2$ is described in JP—A—79143516.

Polymeric materials have also been reported to be effective solid or gelled stabilising agents. For example, polyvinyl pyrrolidone is disclosed as a stabiliser for prostaglandin E compounds in US—A—3826823, polyethylene oxide cross-linked with urethane groups is described for prostaglandin formulation in EP—A—0016652 and EP—A—0016654, and optionally cross-linked carbohydrate polymers are described in BE—A—0881351.

Colloidal silica is disclosed as a gelling agent for organic liquids in C.A. *83* (1975) 183331q (1975). Sheriff *et al*, J. Pharm, Sci., *68(7)* (1979) 842—5, describe the use of colloidal silica dioxide with organic solvents for preparing gels containing pharmaceutically active materials. C.A. *94(12)* (1981) 9033a, describes a stable mixture of $PGE_1$ and kaolin.

According to the invention, a pharmaceutically-acceptable composition, in the form of a free-flowing gel, comprises a PGE compound, an organic liquid, and colloidal silicon dioxide (CSD).

The PGE compound may be any of those exemplified above. The organic solvent is preferably a glyceryl ester which is preferably triacetin. When triacetin is used, the amount of CDS is preferably from 3 to 15, most preferably about 8, % by weight of the composition. Lesser concentrations may result in gels of lesser viscosity, which may not be sufficiently elegant in actual clinical application. Gels which contain more than 8% CSD may be too viscous, and of reduced extendability; since such gels tend to increase in viscosity upon storage, they are especially suitable for use promptly following formulation.

The stable dosage forms provided in accordance with the present invention are used for the same purposes and by the same methods as the known compositions of PGE compounds in a pharmaceutically-acceptable stabilising organic solvent, e.g. as described in US—A—3966962. However, the novel forms are more adaptable for formulation and use, by virtue of being in gel form. For example, when used for intravaginal, trans-cervical, intra-cervical or extra-amniotic injection, the gel is prepared under sterile conditions

and administered via syringe. The present invention provides gels having physical characteristics, at ambient temperature, which permit free and unobstructed flow when so administered.

A novel composition containing $PGE_2$, in a viscous gel, is advantageously used to effect cervical ripening by extra-amniotic administration, the methodology being described by Calder *et al, supra.*

The present invention provides stabilised gels which can be manufactured under sterile conditions, and then packaged and distributed to hospitals or other users without the need for reconstitution, i.e. the addition of water, prior to use (as has been necessary for known gels). Particularly, a novel composition containing $PGE_2$ can exhibit a prolonged shelf-life, i.e. greater than one year, during storage at ambient temperature. The novel gels can maintain their physical integrity more completely upon administration than known hydrophilic gels; therefore, unlike the known gels, they can be withdrawn if necessary from the patient following extra-amniotic, trans-cervical application.

In preparing a gel of the invention, the PGE compound tend to increase in viscosity upon storage, percentages greater than 8 are especially useful when gels are to be used promptly following formulation.

The stable dosage forms provided in accordance with the present invention are used for the same purposes and by the same mothod as the prior art PGE compounds in a pharmaceutically acceptable stabilising organic solvent with the difference that the present forms are rendered more adaptable for formulation and use by virtue of being in the gel form. Accordingly, dosage forms in accordance with the present invention are used for various purposes described in U.S. Patent 3,966,962, incorporated here by reference. However, the present gel composition are particularly advantageous in that these materials are more readily handled and formulated than liquids. Moreover, for example, when used for intravaginal, transcervical or intracervical, or extraamniotic injection, the gel is prepared under sterile conditions and administered via syringe. Advantageously, the present invention provides gels whose physical characteristics and ambient temperature permit free and unobstructed flow when so administered.

More particularly, the present invention provides a highly advantageous method of effecting cervical ripening utilizing extraamniotic administration of $PGE_2$ in a viscous gel. The methodology of employing $PGE_2$ in such viscous gels for extraamniotic cervical ripening is described in Calder, A. A., et al., British Journal of Obstetrics and Gynecology 84:264—268 (1977), cited above.

Unlike prior art gels which required reconstitution prior to use, the present invention provides stabilized gels which can be manufactured under sterile conditions, packaged and distributed to hospitals or other users without the need for reconstitution, i.e., the addition of water, prior to use. Particularly, prostaglandin $E_2$ prepared in the novel dosage form of the present invention can exhibit a prolonged shelf-life, i.e., greater than one year, during storage at ambient temperature. Further, unlike hydrophilic gels of the prior art the present gels can maintain the physical integrity of the gel more completely upon administration and therefore, unlike these prior art gels, be withdrawn if necessary from the patient following extraamniotic, transcervical application.

In preparing the gels of the present invention, the PGE compound is first dissolved in the selected organic solvent by conventional means. For example, the dissolution of $PGE_2$ and triacetin can be readily achieved using a mixer fitted with a small disintegrating head. During the mixing process, which usually runs to completion over the course of several minutes, cooling may be required in order to prevent heat generated by the mixing process from decomposing the $PGE_2$.

Thereafter the colloidal silicon dioxide is incorporated into the resulting solution by combining these two ingredients in a single vessel and mixing with a stirrer. Gelling under these conditions is orginarily effected over the course of several minutes.

The preparation of sterile product can be accomplished by an ultrafiltration of the PGE compound in the organic solvent into a sterile area. Subsequently the colloidal silica dioxide can be heat sterilized and incorporated into the sterilized PGE compound solution.

In accordance with the present invention, pharmaceutical grades of both the organic solvent and colloidal silicon dioxide must be selected. For example, when triacetin is the selected organic solvent, USP or food grade quality material, particularly material known to be low in heavy metal compound content, is readily employed. Moreover, employing triacetin low in metal ion content results in improved stability of the resulting PGE gel.

Similarly, colloidal silica dioxide is available as a pharmaceutical grade material. For example, Cabot Corporation markets CAB-O-SIL M5 colloidal silica dioxide, a material used in oral pharmaceutical formulations of prostaglandin $E_2$ (PROSTIN E2 compressed tablets, Upjohn). Similarly AEROSIL 200 and AEROSIL FK 320 are similar grades of colloidal silicon dioxide marketed by Dugussa.

Since some organic solvents are incompatible with the use of plastic packaging material, e.g., polypropylene syringes, ordinarily a glass syringe is employed and a preselected amount of the gel placed there within. However, when triacetin is employed as the organic solvent, the preferred syringe is a plastic syringe, e.g., polypropylene or high density polyethylene. The use of such plastic syringes is preferred in order to avoid the leaching of impurities into the formulation from the rubber-containing parts of glass syringes.

Alternatively, conventional gelatin capsules may be utilized in formulating these gels into their furnished pharmaceutical form. For example hard gelatin capsules are especially useful.

The pharmaceutically acceptable stabilizing organic liquid or solvents utilized in accordance with the present invention are those which are relatively non-toxic and non-irritant to body tissues which they

contact and those having a capacity to stabilize PGE compounds when placed in solution therewith. One especially useful group of materials for this purpose are glyceryl esters. Such compounds are either mono-, bis-, or tris- esters of glycerol and are known in the art and prepared by methods readily known in the art. For example, triacetin is one such substance. Dipolar aprotic solvents, such as DMA referred to above, are also useful liquids for this purpose. Similarly, other polyol esters such as triethyl citrate are known to be useful in pharmaceutically acceptable stabilizing organic liquids for use in accordance in the present invention.

Alcohols represent another, although less preferred pharmaceutically acceptable stabilizing organic liquid inasmuch as the lower polyols, such as ethanol, are subject to evaporative loss of volume thereby complicating unnecessarily formulation of the resulting gel. Accordingly, useful pharmaceutically acceptable stabilizing organic liquids or solvents utilized in accordance with the present invention include numerous known organic solvents for the stabilization of prostaglandin all of the following chemical compounds.

The stabilizing liquid to be employed in the present invention must also be pharmaceutically acceptable in the sense that the quantities employed must be essentially non-toxic and well tolerated by the animal or patient being treated. The examples above describe such liquids. Organic solvents such as benzene are, for example, unsuitable for reasons of toxicity.

The amount of colloidal silica dioxide employed in any gel depends on the nature of the organic liquid employed. However, in general, the minimum amount of colloidal silicon dioxide necessary is that required to give necessary minimum viscosity to the selected liquid. Ordinarily, gels are preferred which are sufficiently viscous to remain in place when administered. Similarly, the maximum percentage by weight of colloidal silicon dioxide desired is that which defines the maximum desired viscosity of the gel and ordinarily limited by the ability of the gel to be extruded through syringes, catheters or similar extrusion apparatus.

By way of illustrative example, gels are prepared with between 0.25 mg and 3.0 mg of $PGE_2$ per 3 g or 2.5 ml of gel. The following ingredients are used:

(a) $PGE_2$ (dinoprostone PROSTIN E2, Upjohn) 0.25 mg—3.09 mg
(b) colloidal silicon dioxide NF 200 mg—300 mg
(c) triacetin USP 2.7 g—2.8 g

$PGE_2$ is dissolved in triacetin using a Silverson mixer fitted with a 12.7 mm disintegrating head. After mixing for 15 minutes using an ice-bath to prevent heat disintegration of $PGE_2$, colloidal silicon dioxide is then incorporated into the $PGE_2$ solution, until the desired viscosity is obtained, by mixing the $PGE_2$/triacetin solution with the appropriate amount of silicon dioxide until the mixture is gelled, i.e. about 2 minutes. The gel may then be packed in 5 ml Hytac SCF glass syringes (Becton Dickinson) which have been washed, silicised and sterilised, in 5 ml of Pharma-Plant polypropylene syringes which have been sterilised by exposure to ethylene oxide, or in empty gelatin capsules of suitable size.

**Claims**

1. A pharmaceutically-acceptable composition, in the form of a free-flowing gel, which comprises a PGE compound, colloidal silicon dioxide and a stabilising organic liquid.
2. A composition according to claim 1, wherein the organic liquid is a glyceryl ester.
3. A composition according to claim 2, wherein the glyceryl ester is triacetin.
4. A composition according to claim 2, which comprises from 8 to 15% by weight of the colloidal silicon dioxide.
5. A composition according to any of claims 1 to 4, wherein the PGE compound is $PGE_2$.
6. A composition according to any of claims 1 to 4, wherein the PGE compound is (15R)-15-methyl-$PGE_2$.
7. A composition according to any of claims 1 to 4, wherein the PGE compound is 16,16-dimethyl-$PGE_2$.
8. A composition according to any of claims 1 to 4, wherein the PGE compound is (17S),20-dimethyl-$PGE_2$.
9. A composition according to any of claims 1 to 4, wherein the PGE compound is 15-methyl-$PGE_2$.
10. A composition according to any of claims 1 to 4, wherein the PGE compound is $PGE_1$.

**Patentansprüche**

1. Eine pharmazeutisch annehmbare Zusammensetzung in der Form eines freifliessenden Gels, welches eine PGE Verbindung, kolloidales Siliciumdioxid und eine stabilisiernde organische Flüssigkeit umfasst.
2. Eine Zusammensetznung nach Anspruch 1, worin die organische Flüssigkeit ein Glycerylester ist.
3. Eine Zusammensetzung nach Anspruch 2, worin das Glycerylester Triacetin ist.
4. Eine Zusammensetzung nach Anspruch 2, die zwischen 8 und 15 Gewichtsprozent des kolloidalen Siliciumdioxids umfasst.
5. Eine Zusammensetzung nach einem der Ansprüche 1 bis 4, worin die PGE Verbindung $PGE_2$ ist.

6. Eine Zusammensetzung nach einem der Ansprüche 1 bis 4, worin die PGE Verbindung (15R)-15-Methyl-$PGE_2$ ist.

7. Eine Zusammensetzung nach einem der Ansprüche 1 bis 4, worin die PGE Verbindung 16,16-dimethyl-$PGE_2$ ist.

8. Eine Zusammensetzung nach einem der Ansprüche 1 bis 4, worin die PGE Verbindung (17S),20-Dimethyl-$PGE_2$ ist.

9. Eine Zusammensetzung nach einem der Ansprüche 1 bis 4, worin die PGE Verbindung 15-Methyl-$PGE_2$ ist.

10. Eine Zusammensetzung nach einem der Ansprüche 1 bis 4 worin die PGE Verbindung $PGE_1$ ist.

**Revendications**

1. Composition acceptable du point de vue pharmaceutique, sous la forme d'un gel s'écoulant librement, qui comprend un composé du type PGE, du dioxyde de silicium colloïdal et un liquide organique stabilisant.

2. Composition suivant la revendication 1, dans laquelle le liquide organique est un ester de glycéryle.

3. Composition suivant la revendication 2, dans laquelle l'ester de glycéryle est la triacétine.

4. Composition suivant la revendication 2, qui comprend 8 à 15% en poids de dioxyde de silicium colloïdal.

5. Composition suivant l'une quelconque des revendications 1 à 4, dans laquelle le composé PGE consiste en $PGE_2$.

6. Compositions suivant l'une quelconque des revendications 1 à 4, dans laquelle le composé PGE consiste en (15R)-15-méthyl-$PGE_2$.

7. Composition suivant l'une quelconque des revendications 1 à 4, dans laquelle le composé PGE consiste en 16,16-diméthyl-$PGE_2$.

8. Composition suivant l'une quelconque des revendications 1 à 4, dans laquelle le composé PGE consiste en (17S),20-diméthyl-$PGE_2$.

9. Composition suivant l'une quelconque des revendications 1 à 4, dans laquelle le composé PGE consiste en 15-méthyl-$PGE_2$.

10. Composition suivant l'une quelconque des revendications 1 à 4, dans laquelle le composé PGE consiste en $PGE_1$.